# EUROPÄISCHE PATENTANMELDUNG

(11) **EP 4 775 162 A1**
(43) Veröffentlichungstag der Anmeldung: **15.07.2026**
(21) Anmeldenummer: 25224584.0
(22) Anmeldetag: 17.12.2025
(51) Int. Cl.: A61B 17/17, A61B 17/74, A61B 17/88

(54) **SET ZUR VERSORGUNG VON KNOCHENBRÜCHEN MIT EINEM H-FÖRMIGEN WERKZEUG**

(30) Priorität: 14.01.2025 AT 500172025
(71) Anmelder: I.T.S. GmbH, 8301 Lassnitzhöhe (AT)
(72) Erfinder: Prager, Ronald, 24796 Bovenau (DE); Resedaritz, Thomas, 8264 Großhartmannsdorf (AT)
(74) Vertreter: Wirnsberger & Lerchbaum Patentanwälte OG

(57) **Zusammenfassung**

Die Erfindung betrifft ein Set (1) zur Versorgung von Knochenbrüchen, insbesondere proximalen Femurfrakturen, umfassend einen Knochennagel (2), eine Knochenschraube (3) und eine Stellschraube, wobei der Knochennagel (2) eine Öffnung (5) aufweist, durch welche die Knochenschraube (3) führbar ist, und wobei die Stellschraube mit der Knochenschraube (3) in einem Berührungsvolumen in Kontakt bringbar ist, um eine Bewegungsfreiheit der Knochenschraube (3) zu bestimmen. Erfindungsgemäß ist vorgesehen, um eine korrekte Ausrichtung der Knochenschraube (3) für die einzugreifende Stellschraube zu erreichen, dass das Set (1) ein Werkzeug (6) zur formschlüssigen Ausrichtung der auf einem Bohrdraht (16) positionierten Knochenschraube (3) relativ zu einem weiteren Bohrdraht (7)umfasst.

Des Weiteren betrifft die Erfindung ein Werkzeug (6) für ein Set (1) der vorstehend genannten Art. Darüber hinaus betrifft die Erfindung eine Kombination aus einem Werkzeug (6) und einem Befestigungsinstrument für eine Knochenschraube (3).

## Beschreibung

Die Erfindung betrifft ein Set zur Versorgung von Knochenbrüchen, insbesondere proximalen Femurfrakturen, umfassend einen Knochennagel, eine Knochenschraube und eine Stellschraube, wobei der Knochennagel eine Öffnung aufweist, durch welche die Knochenschraube führbar ist, und wobei die Stellschraube mit der Knochenschraube in einem Berührungsvolumen in Kontakt bringbar ist, um eine Bewegungsfreiheit der Knochenschraube zu bestimmen.

Des Weiteren betrifft die Erfindung ein Werkzeug für ein Set zur Versorgung von Knochenbrüchen, insbesondere proximalen Femurfrakturen, wobei das Set einen Knochennagel, eine Knochenschraube und eine Stellschraube umfasst, wobei die Knochenschraube und die Stellschraube durch eine Öffnung des Knochennagels führbar sind, um in einem Berührungsvolumen miteinander zu interagieren.

In einem noch weiteren Aspekt der Erfindung betrifft diese eine Kombination aus einem Werkzeug der vorstehend genannten Art und einem Befestigungsinstrument für eine Knochenschraube.

Schließlich betrifft die Erfindung ein Operationsverfahren zur Versorgung von Knochenbrüchen, insbesondere proximalen Femurfrakturen, wobei insbesondere ein Set und/oder ein Werkzeug der vorstehend genannten Art eingesetzt wird.

Proximale Femurfrakturen sind komplizierte Frakturen, die insbesondere bei älteren Personen auftreten. Problematisch ist insbesondere, dass neben der Komplexität der Frakturen eine Knochenheilung aufgrund des durchschnittlichen Patientenalters lange dauert und daher eine besonders exakte, aber auch flexible Versorgung einer Fraktur wünschenswert ist. Neben relativ einfachen Fixationssystemen sind in den letzten Jahren fortschrittlichere Systeme zur Versorgung entsprechender Oberschenkelhalsbrüche bekannt geworden, welche neben einem notwendigen Knochennagel, insbesondere einem intermedullären Nagel, eine Knochenschraube und eine mit der Knochenschraube zusammenwirkende bzw. interagierende Stellschraube umfassen. Die Stellschraube kann in einfachen Ausführungen derart an die Knochenschraube angreifen, dass die Knochenschraube nach deren Fixierung in einem Femurkopf in Bezug auf eine Rotation, aber auch eine laterale Verschiebbarkeit und ebenso eine mediale Verschiebbarkeit fixiert ist. Bekannt sind allerdings auch Systeme der Anmelderin, in welchen neben einer Rotationsstabilität durch Interaktion der Stellschraube mit der Knochenschraube eine vorbestimmte laterale Bewegbarkeit der Knochenschraube gegeben ist, die entsprechend einer Anstellung der Stellschraube bzw. einem Anschlag der Knochenschraube an der Stellschraube vorbestimmt ist bzw. vorbestimmt werden kann. Auch sind in diesem Zusammenhang Systeme bekannt, in welchen eine gewünschte laterale Verschiebung auf die Knochenschraube vor dem Heilungsprozess bzw. unmittelbar während der Operation initiiert und damit eine Kompression auf die Frakturstücke, insbesondere Femurkopf und/oder Oberschenkelhals und Oberschenkelknochen, aufgebracht werden kann, sodass ein Femurkopf bewusst Richtung lateral gezogen werden kann, um Knochenteile zu komprimieren.

Ein Set der eingangs genannten Art mit einer Knochenschraube sowie einer Stellschraube, neben einem Knochennagel, kann dazu ausgelegt sein, eine Oberschenkelhalsfraktur dynamisch zu versorgen, indem die Stellschraube geeignet geometrisch auslegt ist, um eine dynamisierte Bewegung der Knochenschraube insbesondere in lateraler Richtung zu ermöglichen. Hierfür weist die Knochenschraube üblicherweise eine oder mehrere längserstreckte Nuten auf, wobei die Stellschraube unter leichter Schrägstellung in eine der Nuten eingreift. Durch den Eingriff der Stellschraube in die Knochenschraube, wobei beide Schrauben, also sowohl Knochenschraube als auch Stellschraube, durch eine Öffnung des Knochennagels geführt sein können, wird zunächst eine Rotation des mit der Knochenschraube fixierten Femurkopfes vermieden. Die Nut bzw. die (mehreren) Nuten stellen ein Berührungsvolumen dar, in welches die Stellschraube eingreifen kann. Wenn die Stellschraube als solche zusätzlich in lateraler Richtung Hindernisse für die Knochenschraube vorsieht, kann in geeigneter geometrischer Abstimmung mit der Knochenschraube auch eine vorbestimmte laterale Bewegung der Knochenschraube und damit des Femurkopfes Richtung Oberschenkelschaft eingestellt werden.

Um eine Knochenschraube und auch eine Stellschraube einzubringen, werden üblicherweise entsprechende Bohrdrähte gesetzt, die parallel verlaufen sollten (superiorer und inferiorer Bohrdraht). Der Prozess zur Versorgung einer proximalen Femurfraktur umfasst somit zunächst die Einbringung eines Knochennagels in den Markraum eines Oberschenkelknochens, wofür ein sogenanntes Zielgerät verwendet wird. Der Knochennagel kann bei Bedarf auch leicht eingehämmert werden. Das Zielgerät ist etwa sichelförmig ausgebildet und weist eine Aufnahme für eine Masterhülse und eine oder mehrere Gewebeschutzhülsen auf, welche nach einem Schnitt des Gewebes eingeführt werden, um etwa quer (üblicherweise in einem Winkel von etwa 100° bis 140°) Führungshülsen für Bohrdrähte für die Knochenschraube selbst (üblicherweise superior angeordneter Bohrdraht) und eine Rotationstabilität beim Einbringen der kanülierten Knochenschraube (inferior angeordneter Bohrdraht) einzubringen. In einem nächsten Schritt werden die entsprechenden Bohrdrähte gesetzt und danach wird zunächst die Knochenschraube in den Femurkopf eingetrieben, wobei der superiore Bohrdraht für die kanülierte Knochenschraube als Führung dient. Die Knochenschraube wird dabei mit einem Befestigungsinstrument wie einem Schraubendreher vorwärtsgetrieben, und zwar durch Rotation. Die medial endseitig mit einem Gewinde ausgestattete Knochenschraube wird dadurch in den Femurkopf eingetrieben. Dabei ist darauf achtzugeben, dass die Knochenschraube derart befestigt wird, dass deren Nut oder gegebenenfalls Nuten (üblicherweise sind beispielsweise vier Nuten in einem Winkel von 90° bezogen auf den Umfang vorgesehen) derart zu liegen kommt, dass zunächst der distal oder inferior für die Stellschraube erforderliche Bohrer während des Bohrens nicht an der Knochenschraube anstößt und letztlich auch die Stellschraube entsprechend in einem Berührungsvolumen zu liegen kommen kann. Ist die Knochenschraube diesbezüglich verdreht, stößt zunächst im Operationsprozess der Bohrer für die Stellschraube bereits an der Knochenschraube an bzw. kann nicht weiterbewegt werden. Selbst wenn der Bohrer noch durchgängig ist, kann im weiteren Operationsprozess eine Situation gegeben sein, dass die Stellschraube nicht perfekt in ein Berührungsvolumen einbringbar ist, welches im Wesentlichen durch eine Nut der Knochenschraube definiert ist.

Um eine besonders genaue Ausrichtung diesbezüglich zu ermöglichen, kann vorgesehen sein, dass das Befestigungsinstrument für die Knochenschraube eine Markierung aufweist und dies auch für eine die einzutreibende Knochenschraube umgebende Hülse, welche auch eine Ausrichtung eines inferioren Bohrdrahtes unmittelbar oder mittelbar festlegt, der Fall ist. Wenn diese beiden Markierungen fluchtend in lateraler Richtung übereinstimmen, ist eine Position gegeben, in welcher die oder eine Nut der Knochenschraube (je nachdem, wie viele vorliegen) so liegen sollte, dass zunächst der Bohrer und anschließend die Stellschraube in distaler Distanz entsprechend geführt werden können, um eine gewünschte Interaktion zwischen Stellschraube und Knochenschraube im Berührungsvolumen erreichen zu können, ohne dass es vorher zu Komplikationen kommt. Allerdings tritt in der Praxis der Fall auf, dass nach der fluchtenden Ausrichtung des Befestigungsinstrumentes mit der korrespondierenden Markierung, beispielsweise an einer Führungshülse, eine Umlagerung der zu operierenden Person oder eine leichte Verschiebung der Gewebeschutzhülse, welche zum Beispiel eine korrespondierende Markierung trägt, erfolgt, sodass die Knochenschraube mit deren Befestigungsvolumen nicht mehr geeignet ausgerichtet ist. Dies kann dazu führen, dass ein Bohrer für die Festlegung eines Loches für die Stellschraube nicht mehr durchgängig durchgeführt werden kann, sondern an der Knochenschraube anstößt oder sich Probleme beim Justieren der Stellschraube ergeben. Dies ist im Operationsprozess unerwünscht, weil dies nicht nur zu längeren Operationszeiten, sondern auch zu Mehraufwand beim Operationspersonal führen kann, weil sich die Situation von einer Normsituation unterscheidet.

Aufgabe der Erfindung ist es, ein an sich bereits sehr gut ausgelegtes Set der eingangs genannten Art derart weiterzubilden, dass auch die vorstehenden Komplikationen verhindert oder zumindest verringert sind.

Ein weiteres Ziel der Erfindung ist es, ein hierfür geeignetes Werkzeug der eingangs genannten Art anzugeben.

Schließlich ist es ein weiteres Ziel der Erfindung, eine Kombination aus einem Werkzeug der eingangs genannten Art und einem Befestigungsinstrument für eine Knochenschraube anzugeben, welche die vorstehend erläuterten Probleme beseitigt oder zumindest verringert.

Die Aufgabe der Erfindung betreffend ein Set wird gelöst, wenn bei einem Set der eingangs genannten Art das Set ein Werkzeug zur formschlüssigen Ausrichtung der auf einem Bohrdraht positionierten Knochenschraube relativ zu einem weiteren Bohrdraht umfasst.

Mit einem derartigen Set wird der Vorteil erreicht, dass die Knochenschraube in Relation zum weiteren durch die Öffnung verlaufenden Bohrdraht und damit auch für die später einzubringende Stellschraube exakt platziert ist. Die Stellschraube kann daher problemlos an- bzw. eingebracht werden und in ein Berührungsvolumen eingreifen, welches insbesondere durch die Knochenschraube definiert wird. Beispielsweise kann das Berührungsvolumen eine Aussparung wie eine Nut oder mehrere am Umfang der Knochenschraube insbesondere gleichmäßig verteilte Nuten, aber auch andere geometrische Formen wie stufenförmige Abkantungen darstellen. Durch die formschlüssige Ausrichtung und Verbindung der Knochenschraube mit dem weiteren (inferioren) Bohrdraht ist noch ohne Einbringung der Stellschraube eine interoperative Rotationsstabilität der Knochenschraube relativ zum Bohrdraht gegeben. Dadurch kann es während dieser formschlüssigen Verbindung selbst bei Umlagerungen von Patienten während einer Operation nicht dazu kommen, dass sich eine manuell zunächst korrekt ausgerichtete Knochenschraube positionell verändert, beispielsweise weil am Patienten hantiert wird oder Faszien des Patienten und Zug- und/oder Druckkräfte derselben zu einem Verrücken der Gewebeschutzhülse führen.

Das Werkzeug verbindet die Knochenschraube und den weiteren (inferioren) Bohrdraht in der Regel mittelbar. Dabei kann insbesondere vorgesehen sein, dass das Set eine Führung, insbesondere eine Hülse, für den weiteren Bohrdraht umfasst, wobei die Führung das Werkzeug zur Ausrichtung der Knochenschraube formschlüssig aufnimmt und umgekehrt. Das Werkzeug greift hierfür in der Regel außenseitig an der Führung an. Für den Gegenpart der formschlüssigen Verbindung kann vorgesehen sein, dass das Set einen Dreher für die Knochenschraube umfasst, wobei das Werkzeug am Dreher verschiebbar, insbesondere linear verschiebbar, lagerbar und/oder gelagert ist. Der Dreher in Form eines zum Beispiel Schraubendrehers ist vorgesehen, um die Knochenschraube über den für die Knochenschraube vorgesehenen Bohrdraht einzuschrauben und dabei über Markierungen eine korrekte Position der Knochenschraube festzulegen, sodass nach Entfernung des inferioren Bohrdrahtes und der Hülse für diesen zunächst eine Führungshülse für den Bohrer in eine Masterhülse und/oder Gewebeschutzhülse eingeführt und der Bohrer für die Stellschraube während eines Bohrens an der Knochenschraube vorbeigeführt werden kann und anschließend die Stellschraube beim Einbringen in Bezug auf die Knochenschraube korrekt zu liegen kommt. Der Dreher kann an dessen medialen Ende endseitig in ein laterales Ende der Knochenschraube eingreifen, insbesondere formschlüssig wie über einen Sechskant. Das Werkzeug kann in diesem Fall am Dreher gelagert sein. Hierfür kann das Werkzeug insbesondere auf den Dreher aufgeschoben werden und ist auf diesem vorteilhafterweise verschiebbar gelagert. Der Dreher kann dann zusammen mit dem aufgeschobenen Werkzeug betätigt werden, um die Knochenschraube in eine gewünschte Position einzuschrauben. Das Werkzeug ist dabei beispielsweise endseitig am Dreher gelagert und inaktiv. Sobald die Knochenschraube in einer nach menschlichem Ermessen und auf Basis der erwähnten Markierungen korrekten Position ist, kann das Werkzeug von einer lateralen Position am Dreher nach medial verschoben werden, sodass das Werkzeug am Dreher einerseits anliegt und an einer Hülse für den inferioren Bohrdraht andererseits zu liegen kommt. Da die Hülse für den inferioren Bohrdraht in einer außenseitig liegenden oder übergreifenden sogenannten Masterhülse im Wesentlichen positionell fixiert vorliegt, sorgt die formschlüssige Verbindung der Hülse mit der Knochenschraube über das Werkzeug für eine mittelbare formschlüssige Verbindung der Knochenschraube mit dem Bohrdraht, wobei die Knochenschraube nicht nur rotationsstabil gehalten wird, sondern auch korrekt positionell ausgerichtet ist. In weiterer Folge kann der Dreher abgenommen und der inferiore Bohrdraht samt Hülse entfernt werden, wonach die erforderliche Bohrung für die Stellschraube erfolgen kann, ehe dann die Stellschraube eingeführt wird, nachdem zuvor der Bohrer entfernt worden ist.

In diesem Zusammenhang ist es besonders bevorzugt, dass das Werkzeug zumindest eine Nase aufweist und der Dreher zumindest eine korrespondierende Vertiefung aufweist, um die Nase in der Vertiefung aufzunehmen. Die zumindest eine Vertiefung kann die Nase formschlüssig aufnehmen. Bevorzugt sind mehrere Nasen und mehrere korrespondierende Vertiefungen vorgesehen. Sind beispielsweise zwei um 180° gegenüberliegende Nasen und entsprechend zwei um 180° gegenüberliegende Vertiefungen am Dreher vorgesehen, kann das Werkzeug optimal am Dreher gleiten und auch leicht aufgesetzt und abgenommen werden. Andere Ausrichtungen mit beispielsweise 90° oder 120° sind selbstverständlich auch möglich, erfordern aber mehr Fertigungsaufwand. Ein falsches Aufsetzen ist nicht möglich, da mehrere Nasen für eine korrekte Positionierung des Werkzeuges am Dreher sorgen.

Um mögliche menschliche Fehlerquellen durch falsche Handgriffe möglichst auszuschließen, ist mit Vorteil vorgesehen, dass das Werkzeug in stirnseitiger Ansicht spiegelsymmetrisch zu einer ersten Mittelachse und/oder spiegelsymmetrisch zu einer zweiten Mitteachse ausgebildet ist. Je höher die Symmetrie, umso weniger anfällig ist das Werkzeug für eine falsche Montage. Idealerweise kann das Werkzeug insbesondere auf einen Dreher nur in einer einzigen, gleichzeitig formschlüssig aktiven Variante aufgesetzt werden, sodass diesbezüglich kein Fehler erfolgen kann.

Das Befestigungsvolumen ist mit Vorteil durch zumindest eine, bevorzugt mehrere, Nuten in der Knochenschraube gebildet. Mehrere Befestigungsvolumina, insbesondere Nuten, an bzw. in der Knochenschraube sind zweckmäßig, damit eine Eindrehtiefe für die Knochenschraube insbesondere in einen Femurkopf flexibel gestaltet werden kann und die Knochenschraube nicht beispielsweise zu weit eingedreht werden muss, damit in eine einzige vorhandene Nut ein Eingriff durch die Stellschraube erfolgen kann.

Mit Vorteil ist an einer Hülse, insbesondere einer Führungshülse, eine erste Markierung angebracht und korrespondiert hierzu eine weitere Markierung, welche an einem Einschraubmittel für die Knochenschraube und/oder der Knochenschraube angebracht ist, wobei bei fluchtender Ausrichtung der Markierungen das Berührungsvolumen für die Stellschraube und die Knochenschraube ausgerichtet sind. Dies entspricht der üblichen Justage per Hand, ehe das erfindungsgemäß vorgesehene Werkzeug zur optimierten Ausrichtung aktiviert wird, insbesondere durch Verschieben eines H-förmigen Werkzeuges, welches auf einem Dreher linear verschiebbar gehalten ist, von einer lateralen (End-)Position nach medial, insbesondere in eine mediale Endposition, um die erläuterte formschlüssige Verbindung herzustellen.

Mit Vorteil ist die Stellschraube schräg zur Knochenschraube durch die dieselbe Öffnung geführt, um im Berührungsvolumen einzugreifen, wobei ein Winkel zwischen der Stellschraube und der Knochenschraube 100° bis 140°, vorzugsweise 105° bis 135°, insbesondere 110° bis 130° beträgt. Die schräge (und damit nicht koaxiale) Anstellung der Stellschraube relativ zur Knochenschraube erlaubt es, dass die Stellschraube in ein Berührungsvolumen in der Knochenschraube eingreift, wobei die entsprechende Positionierung auch so gestaltet sein kann, dass die Knochenschraube während des Heilungsprozesses nach lateral bewegbar ist, bis diese an der Stellschraube anschlägt, beispielsweise durch eine nach medial verjüngte Ausbildung der Nut. Bewegt sich die Knochenschraube während des Heilungsprozesses nach lateral und liegt die Stellschraube im Berührungsvolumen, ohne jedoch die Knochenschraube zunächst zu berühren, kann die Knochenschraube solange nach lateral wandern, bis letztlich die Verjüngung der Nut dafür sorgt, dass die Knochenschraube an der Stellschraube anliegt und eine weitere laterale Bewegung der Knochenschraube verhindert wird. Ungeachtet dessen ist aufgrund der Präsenz insbesondere eines Endes der Stellschraube im Berührungsvolumen während des Heilungsprozesses auch eine Rotation der Knochenschraube verhindert, da dies durch die Stellschraube blockiert wird.

Die Stellschraube weist üblicherweise ein Außengewinde auf und in der Öffnung kann ein Innengewinde zur Aufnahme des Außengewindes der Stellschraube vorgesehen sein. Damit kann die Stellschraube in der Öffnung des Knochennagels positionell fixiert gehalten werden. Die Knochenschraube hingegen ist über ein Außengewinde in einem Knochenteil wie einem Femurkopf befestigt, weist allerdings im Bereich der Öffnung kein Außengewinde und im Bereich der Führung durch die Öffnung dieselbe auch kein Innengewinde auf, sodass die Knochenschraube gleitbar in der Öffnung des Knochennagels gelagert ist bzw. in dieser gleiten kann.

In einem weiteren Aspekt betrifft die Erfindung ein Werkzeug der eingangs genannten Art, wobei das Werkzeug in einer stirnseitigen Ansicht eine Führungsöffnung aufweist, sodass das Werkzeug auf ein Befestigungsinstrument für die Knochenschraube wie einen Dreher aufbringbar ist. Mit einem derartigen Werkzeug kann auf einfache Weise sichergestellt werden, dass bei Einbringen einer Knochenschraube dieselbe korrekt positioniert und stabil gehalten werden kann, bis an anderer Position Arbeiten erforderlich sind, insbesondere Arbeiten zum Einbringen eines Bohrers für das spätere Einbringen einer Stellschraube.

Das Werkzeug kann in einer stirnseitigen Ansicht U-förmig oder vorzugsweise H-förmig ausgebildet sein. Eine hohe Symmetrie stellt sicher, dass während einer Stressphase bei einer Operation kein Hantierungsfehler durch falsches Aufsetzen des Werkzeuges auf einen Schraubendreher passieren kann.

Entsprechend den vorstehenden Vorteilen eines erfindungsgemäßen Sets sowie eines hierfür ausgelegten Werkzeuges umfasst die Erfindung in einem weiteren Aspekt eine Kombination aus einem erfindungsgemäßen Werkzeug und einem Befestigungsinstrument für eine Knochenschraube, wobei das Befestigungsinstrument eingerichtet ist, das Werkzeug im Bereich der Führungsöffnung formschlüssig aufzunehmen. Insbesondere kann das Werkzeug am Befestigungsinstrument linear verschiebbar lagerbar oder gelagert sein.

In einem weiteren Aspekt betrifft die Erfindung ein Operationsverfahren der eingangs genannten Art, wobei ein erfindungsgemäßes Set und/oder ein erfindungsgemäßes Werkzeug eingesetzt wird.

In einem weiteren Aspekt betrifft die Erfindung ein Operationsverfahren zur Versorgung von Knochenbrüchen, insbesondere proximalen Femurfrakturen, wobei eine in einen Knochen, insbesondere Femurkopf, eingebrachte Knochenschraube mit einem Werkzeug durch ein formschlüssiges Eingreifen des Werkzeuges an einer Führung für einen weiteren Bohrdraht bei gleichzeitiger Verbindung des Werkzeuges mit der Knochenschraube ausgerichtet wird. Der weitere Bohrdraht ist dabei verschieden von einem Bohrdraht für die Knochenschraube. Insbesondere kann es sich um einen in Relation zum Bohrdraht für die Knochenschraube inferior angeordneten Bohrdraht handeln. Somit wird die Knochenschraube über den superior angeordneten Bohrdraht eingebracht und dann an dem vorzugsweise parallel verlaufenden Bohrdraht bzw. einer Führung für denselben ausgerichtet. Dadurch kann sichergestellt werden, dass die Knochenschraube auch dann geeignet für eine Interaktion mit einer nachfolgend eingebrachten Stellschraube positioniert ist, wenn die Führung für die Knochenschraube nicht ideal positioniert ist. Eine korrekte Ausrichtung ist insbesondere dann erforderlich, wenn die Stellschraube außenseitig an der Knochenschraube angreift, insbesondere wenn die Stellschraube mit einem Winkel und somit nicht koaxial an die Knochenschraube angreift und gegebenenfalls in eine Nut der Knochenschraube eingreift.

Insbesondere kann vorgesehen sein, dass eine Verbindung des Werkzeuges mit der Knochenschraube mittelbar erfolgt, insbesondere über einen Schraubendreher für die Knochenschraube. Der Schraubendreher kann mit einem medialen Ende desselben in Eingriff mit einem lateralen Ende der Knochenschraube gebracht werden, wobei das Werkzeug am Schraubendreher gelagert und zur Ausrichtung der Knochenschraube aus einer lateralen Position in eine mediale Position, jeweils am Schraubendreher, verschoben wird. Dabei kann die Ausrichtung der Knochenschraube durch Rotation der Knochenschraube erfolgt. Die Rotation der Knochenschraube erfolgt dabei um deren Längsachse.

Allgemein kann das zuvor dargestellte Operationsverfahren mehrere oder alle der folgenden Schritte umfassen, wobei die Schritte bevorzugt in der angeführten Reihenfolge durchgeführt werden:
- Verbinden und Einbringen des Knochennagels mit dem Zielgerät;
- Anordnen einer Masterhülse;
- Einbringung einer Führung für einen superioren Bohrdraht und einer Führung für einen inferioren Bohrdraht, wonach die Bohrdrähte, bevorzugt parallel, eingebracht werden;
- Vorbohren für eine kanülierte Knochenschraube, insbesondere eine Knochenschraube mit außenseitigen Nuten;
- Einbringen der Knochenschraube über den superioren Bohrdraht, insbesondere mit einem lateral an der Knochenschraube befestigten Schraubendreher;
- Ausrichten der Knochenschraube relativ zum inferioren Bohrdraht;
- Entfernen des inferioren Bohrdrahtes;
- Einbringen einer inferioren Führungshülse für einen Bohrer zum Bohren eines Lochs für eine Stellschraube;
- Vorbohren für die Stellschraube;
- Entnahme des Bohrers und Einbringen der Stellschraube, wobei die Stellschraube insbesondere in eine Nut der Knochenschraube eingreift.

Weitere Merkmale, Vorteile und Wirkung der Erfindung ergeben sich aus dem nachfolgend dargestellten Ausführungsbeispiel. In den Zeichnungen, auf welche dabei Bezug genommen wird, zeigen:
Fig. 1 ein erfindungsgemäßes Set mit einem Werkzeug in einer ersten, lateralen Position des Werkzeuges;
Fig. 2 das Set aus Fig. 1 in einer rückseitigen Ansicht;
Fig. 3 das Set aus Fig. 1 und Fig. 2 in einer perspektivischen Ansicht;
Fig. 4 das Set aus Fig. 1 bis 3 mit dem Werkzeug in einer medialen Position;
Fig. 5 das Set aus Fig. 4 in einer rücksichtigen Ansicht;
Fig. 6 das Set aus Fig. 4 und Fig. 5 in einer stirnseitigen Ansicht;
Fig. 7 das Set aus Fig. 4 bis Fig. 6 in einer perspektivischen Ansicht;
Fig. 8 eine stirnseitige Ansicht eines Werkzeuges für ein erfindungsgemäßes Set;
Fig. 9 eine Seitenansicht des Werkzeuges aus Fig. 8;
Fig. 10 eine Draufsicht auf ein Werkzeug gemäß Fig. 8;
Fig. 11 eine perspektivische Ansicht eines Werkzeuges gemäß den Fig. 8 bis 10;
Fig. 12 eine schematische Darstellung eines befestigten Knochennagels mit Knochenschraube und Stellschraube.

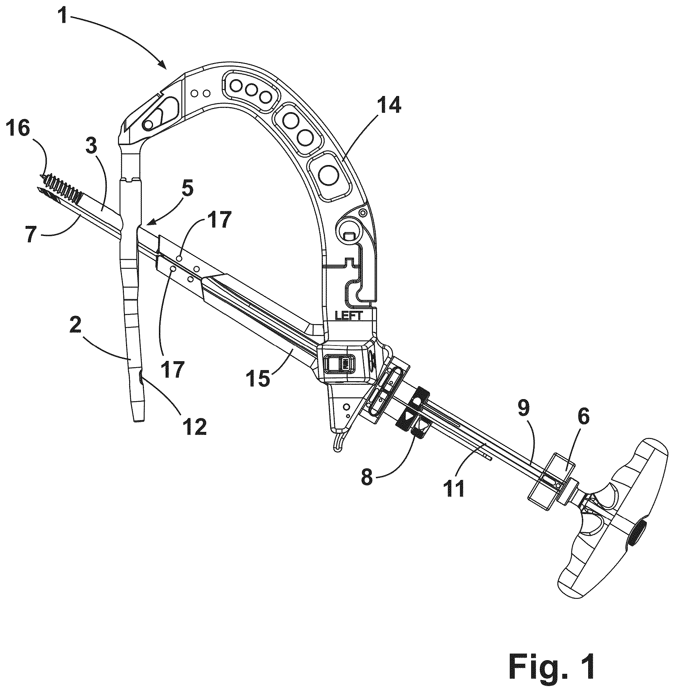

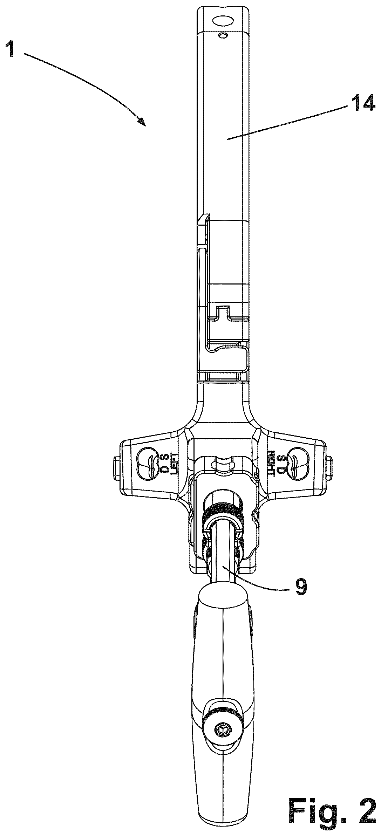

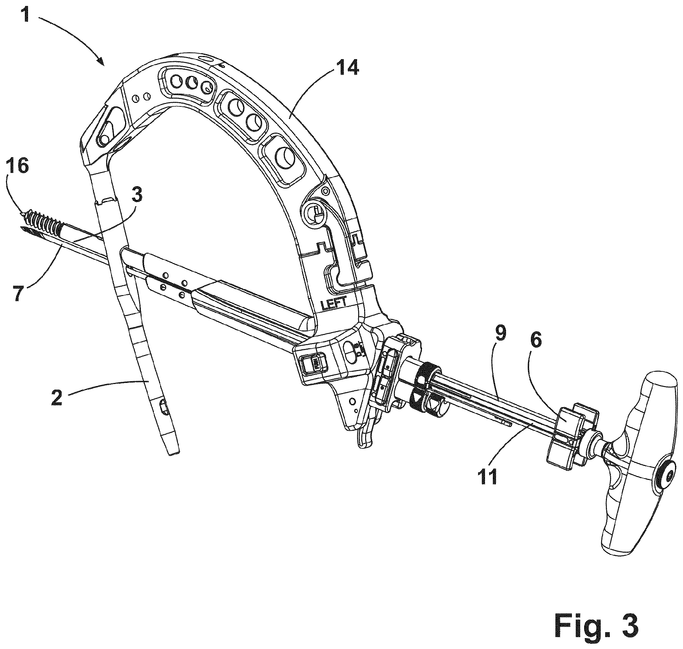

In Fig. 1 bis Fig. 3 ist in verschiedenen Ansichten ein erfindungsgemäßes Set 1 dargestellt. Das Set 1 umfasst ein Zielgerät 14. Das Zielgerät 14 kann mit einem ersichtlichen Knochennagel 2 verbunden werden, um einerseits den Knochennagel 2 insbesondere in einen Oberschenkelknochen bei einer Oberschenkelhalsfraktur einführen zu können. Des Weiteren nimmt das Zielgerät 14 auch dem Knochennagel 2 lateral gegenüberliegend eine Masterhülse 15 auf, die insbesondere auch zur weiteren Aufnahme einer oder mehrerer Gewebeschutzhülsen dient. Die Masterhülse 15 ist durch eine Durchbrechung des Zielgerätes 14 geführt, sodass die Masterhülse 15 zumindest im Wesentlichen in Richtung einer Öffnung 5 des Knochennagels 2 gerichtet ist. Dadurch kann sichergestellt werden, dass später noch einzubringende Teile grundsätzlich richtig positioniert werden. Innerhalb der Masterhülse 15 und gegebenenfalls einer Gewebeschutzhülse befinden sich zunächst eine (in Fig. 1 nicht mehr ersichtliche) Führung für einen Bohrdraht 16 und eine Führung 8 für einen parallel dazu verlaufenden weiteren Bohrdraht 7. Der Bohrdraht 16 stellt eine Führung für eine Knochenschraube 3 dar, welche eine kanülierte Knochenschraube 3 darstellt und über den Bohrdraht 16 geführt ist. Sowohl die Führung 8 als auch die andere Führung können jeweils als einzuschiebende Hülsen ausgebildet sein. Über den Bohrdraht 16 wird die Knochenschraube 3 eingeführt bzw. insbesondere in einen Femurkopf eingetrieben. Unabhängig von einem insbesondere in Fig. 1 und Fig. 3 ersichtlichen Werkzeug 6, welches sich in Fig. 1 bis Fig. 3 in einer lateralen Position, insbesondere in einer lateralen Endposition, an einem Dreher 9 in Form eines Schraubendrehers für die Knochenschraube 3 befindet, stellt die Situation gemäß Fig. 1 bis Fig. 3 eine Situation in einem typischen Operationsverlauf dar. Der Operationsverlauf ist allgemein und losgelöst von dem konkreten Ausführungsbeispiel auch ohne das Werkzeug 6 im Grundsatz wie folgt:
- Verbinden und Einbringen des Knochennagels 2 mit dem Zielgerät 14;
- Anordnen der Masterhülse 15 und optional Gewebeschutzhülsen;
- Einbringung einer Führung für den superioren Bohrdraht 16 und einer Führung 8 für den inferioren Bohrdraht 7, wobei die Bohrdrähte 7, 16 bevorzugt parallel eingebracht werden;
- Vorbohren für die kanülierte Knochenschraube 3;
- Einbringen der Knochenschraube 3 über den Bohrdraht 16 mit einem lateral an der Knochenschraube 3 befestigten Schraubendreher;
- Entfernen des inferioren Bohrdrahtes 7;
- Einbringen einer inferioren Führungshülse für einen Bohrer zum Bohren eines Lochs für die Stellschraube 4;
- Vorbohren für eine Stellschraube 4;
- Entnahme des Bohrers und Einbringen der Stellschraube 4.

Hinzu kommen die üblichen Schritte, die erforderlich sind, um den jeweils nächsten Schritt durchführen zu können, beispielsweise die Entfernung einzelner Führungen 8. Über Fenster 17 kann dabei eine Ausrichtung der Bohrdrähte 7, 16 kontrolliert werden.

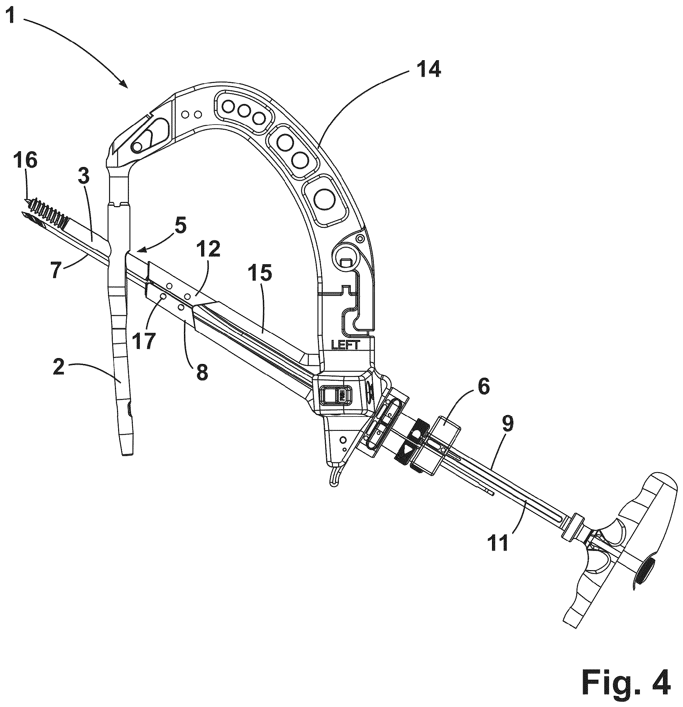

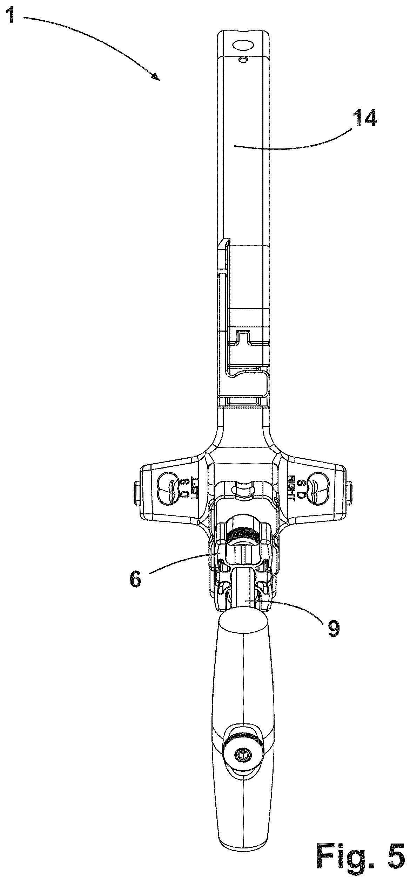

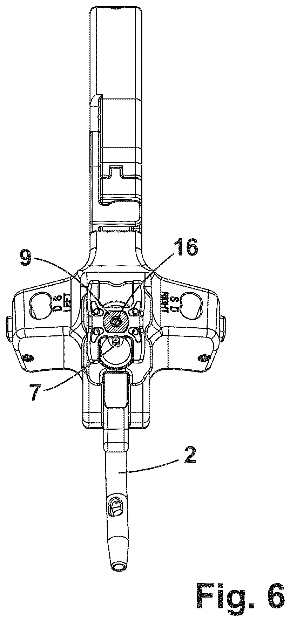

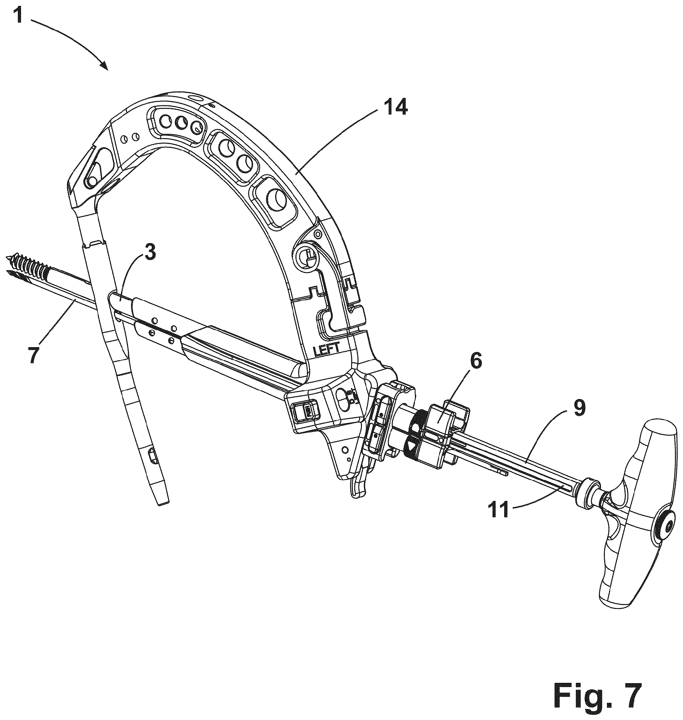

Beim Einbringen der Knochenschraube 3 ist es erforderlich, insbesondere wenn diese mit seitlichen Nuten zur Definition eines Berührungsvolumens für eine Stellschraube 4 ausgebildet ist (zum Beispiel vier außenseitige Nuten, die insbesondere äquidistant um 90° versetzt am Umfang verteilt sind), dass die Knochenschraube 3 richtig positioniert ist. Ist dies nämlich nicht der Fall, kann der Bohrer für die Stellschraube 4 nicht geeignet an der Knochenschraube 3 vorbeigeführt werden, sondern stößt an der Knochenschraube 3 an. Aus diesem Grund weist der Dreher 9 bzw. Schraubendreher für die Knochenschraube 3 eine, insbesondere geradlinige, Markierung auf, welche in Übereinstimmung mit einer Markierung an einer in der Masterhülse 15 sitzenden Gewebehülse oder einem anderen Teil des Sets 1 zu bringen ist. Die Markierung kann nicht nur am Schraubendreher, sondern alternativ oder ergänzend auch an der Knochenschraube 3 angeordnet sein. Bevorzugt sind die zur Übereinstimmung zu bringenden Markierungen Geraden, sodass bei Übereinstimmung eine fluchtende Anordnung der Markierungen gegeben ist. Es kann dann mit relativ hoher Wahrscheinlichkeit davon ausgegangen werden, dass der manuelle Einstellvorgang der Positionierung der Knochenschraube 3 geeignet ist, um zunächst den Bohrvorgang für die Stellschraube 4 vorzunehmen und anschließend die Stellschraube 4 geeignet positionieren zu können. Allerdings kann es im Zuge einer Operation beispielsweise durch Umlagerung des Patienten oder aufgrund kleinerer Verrückungen einer Führungshülse, welche ein geringes Spiel von wenigen Grad aufweist, dazu kommen, dass die an sich korrekte Positionierung der Knochenschraube 3 relativ zum Bohrdraht 7 nicht mehr gegeben ist. Um dies zu verhindern, ist ein Werkzeug 6 vorgesehen, welches in Fig. 1 bis Fig. 3 in einer lateralen Position, insbesondere einer lateralen Endposition, am Dreher 9 verharrt, bis mit dem Dreher 9 die Knochenschraube 3 in eine gewünschte Position (Übereinstimmung der Markierungen) gebracht ist. Später während der Operation wird das Werkzeug 6 am Dreher 9 linear nach medial verschoben, bis das Werkzeug 6 die Führung 8 umfasst, wie dies in Fig. 4 bis Fig. 7 dargestellt ist. Dadurch ist eine formschlüssige Verbindung gegeben und ist die Knochenschraube 3 relativ zum Bohrdraht 7 korrekt ausgerichtet. Somit ist das Risiko minimiert, dass wegen einer Umlagerung des Patienten oder aufgrund anderer manueller Techniken eine Ausrichtung der Knochenschraube 3 relativ zum Bohrdraht 7 und damit letztlich bzw. in weiterer Folge auch zur Stellschraube 4 verloren geht. Nach der korrekten Einstellung kann das Werkzeug 6 abgenommen und der inferiore Bohrdraht 7 samt zugehöriger Hülse entfernt werden. Es kann nun der Bohrer für die Stellschraube 4 geführt werden, wonach die Stellschraube 4 eingeführt werden kann.

Das Verfahren, wie zuvor allgemein definiert, umfasst somit erfindungsgemäß im Allgemeinen zusätzlich den Schritt des Verschiebens des Werkzeuges 6 zur korrekten Ausrichtung der Knochenschraube 3 nach deren Einbringung, aber vor dem Bohren eines Lochs für die Stellschraube 4.

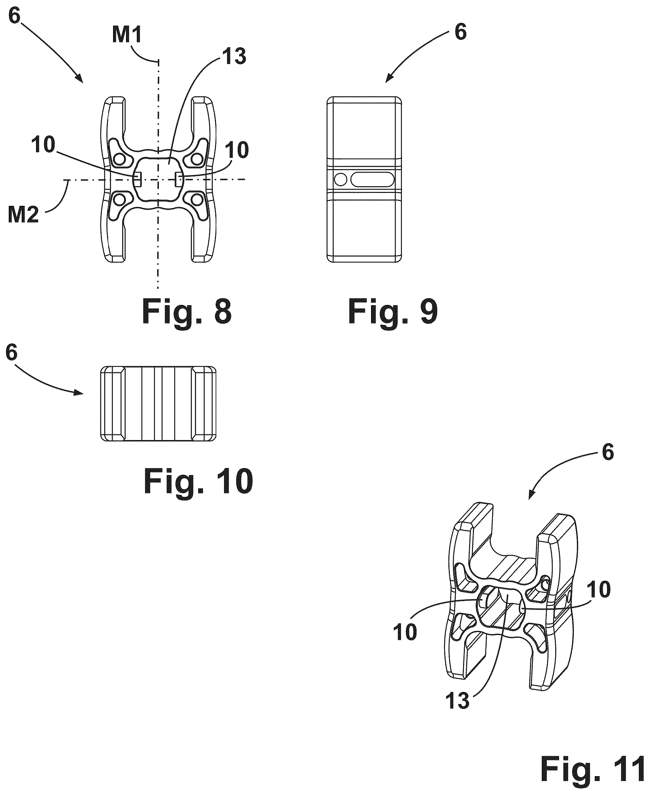

In Fig. 8 bis Fig. 11 ist ein erfindungsgemäßes Werkzeug 6 gesondert in verschiedenen Ansichten dargestellt. Wie insbesondere aus Fig. 8 und Fig. 11 ersichtlich ist, ist das Werkzeug 6 im Wesentlichen H-förmig ausgebildet. Das Werkzeug 6 weist eine hohe Symmetrie auf. Das Werkzeug 6 ist spiegelsymmetrisch zu einer ersten Mittelachse M1 und zu einer zweiten Mittelachse M2 ausgebildet. Somit ergibt sich eine zweizählige Rotationssymmetrie um die entsprechenden Achsen (symmetrische Drehachse von 180°), was dazu führt, dass das Werkzeug 6 an einem Dreher 9 nicht falsch montiert werden kann. Egal, wie das Werkzeug 6 auf einen Dreher 9, wie dieser in Fig. 1 bis Fig. 7 ersichtlich ist, aufgesetzt wird, ist das Werkzeug 6 für oder während einer Operation richtig montiert.

Das Werkzeug 6 weist mit Blick auf Fig. 8 oder Fig. 11 eine zentrale Führungsöffnung 13 auf. Die zentrale Führungsöffnung 13 weist innenseitig zwei Nasen 10 auf, die auf gleicher Höhe angeordnet sind und in entsprechende Vertiefungen 11 eines Befestigungsmittels wie einen Dreher 9 eingeschoben werden können. Dadurch ist einerseits eine formschlüssige Aufnahme des Werkzeuges 6 am Befestigungsmittel wie einem Dreher 9 möglich und kann andererseits ein lineares Verschieben des Werkzeuges 6 am Dreher 9 erfolgen, sodass der Dreher 9 betätigt werden kann, wenn das Werkzeug 6 noch in einer passiven Situation verharrt, wie dies in Fig. 1 bis Fig. 3 dargestellt ist. Ausgehend von der zentralen Führungsöffnung 13 erstrecken sich in Fig. 8 und Fig. 11 sowohl nach oben nach als auch nach unten jeweils zwei Arme, welche die H-förmige Ausbildung des Werkzeuges 6 im Wesentlichen definieren. Die entsprechenden vier Arme (zwei Arme oben, zwei Arme unten) sorgen dafür, dass das Werkzeug 6 in der erläuterten Weise mit anderen Teilen des erfindungsgemäßen Sets 1 derart zusammenwirken kann, dass bereits während der Durchführung einer Operation eine rotationsstabile Ausrichtung einer Knochenschraube 3 relativ zu insbesondere einem Bohrdraht 7 getroffen werden kann, sodass während der Operation sichergestellt ist, dass ein späteres Einbringen einer Stellschraube 4 ohne Komplikationen möglich ist.

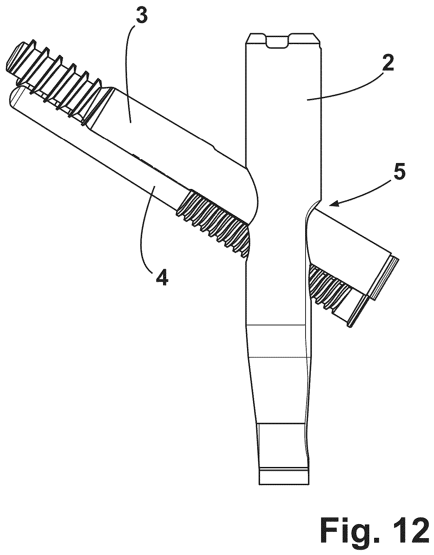

In Fig. 12 ist schließlich eine schematische Darstellung eines befestigten Knochennagels 2 mit Knochenschraube 3 und Stellschraube 4 gezeigt, wie diese nach Durchführung einer Operation und während eines Heilungsprozesses gegeben ist. Zusätzlich ist üblicherweise zumindest eine feststehende distale Befestigung des Knochennagels 2 über in die in Fig. 1 ersichtliche distale Öffnung 12 gegeben, wofür eine weitere Schraube vorgesehen ist, die mit Bezug auf Fig. 12 im Wesentlichen horizontal verläuft.

## Patentansprüche

1. Set (1) zur Versorgung von Knochenbrüchen, insbesondere proximalen Femurfrakturen, umfassend einen Knochennagel (2), eine Knochenschraube (3) und eine Stellschraube (4), wobei der Knochennagel (2) eine Öffnung (5) aufweist, durch welche die Knochenschraube (3) führbar ist, und wobei die Stellschraube (4) mit der Knochenschraube (3) in einem Berührungsvolumen in Kontakt bringbar ist, um eine Bewegungsfreiheit der Knochenschraube (3) zu bestimmen, **dadurch gekennzeichnet, dass** das Set (1) ein Werkzeug (6) zur formschlüssigen Ausrichtung der auf einem Bohrdraht (16) positionierten Knochenschraube (3) relativ zu einem weiteren Bohrdraht (7) umfasst.

2. Set (1) nach Anspruch 1, **dadurch gekennzeichnet, dass** das Werkzeug (6) die Knochenschraube (3) und den weiteren Bohrdraht (7) mittelbar verbindet.

3. Set (1) nach Anspruch 1 oder 2, **dadurch gekennzeichnet, dass** das Set (1) eine Führung (8), insbesondere Hülse, für den weiteren Bohrdraht (7) umfasst, wobei die Führung (8) das Werkzeug (6) zur Ausrichtung der Knochenschraube (3) formschlüssig aufnimmt.

4. Set (1) nach einem der Ansprüche 1 bis 3, **dadurch gekennzeichnet, dass** das Set (1) einen Dreher (9) für die Knochenschraube (3) umfasst, wobei das Werkzeug (6) am Dreher (9) verschiebbar, insbesondere linear verschiebbar, lagerbar und/oder gelagert ist.

5. Set (1) nach Anspruch 4, **dadurch gekennzeichnet, dass** das Werkzeug (6) zumindest eine Nase (10) aufweist und der Dreher (9) zumindest eine korrespondierende Vertiefung (11) aufweist, um die Nase (10) in der Vertiefung (11) aufzunehmen.

6. Set (1) nach Anspruch 5, **dadurch gekennzeichnet, dass** das zumindest eine Vertiefung (11) die Nase (10) formschlüssig aufnimmt.

7. Set (1) nach Anspruch 5 oder 6, **dadurch gekennzeichnet, dass** mehrere Nasen (10) und korrespondierende Vertiefungen (11) vorgesehen sind.

8. Set (1) nach einem der Ansprüche 1 bis 7, **dadurch gekennzeichnet, dass** das Werkzeug (6) in stirnseitiger Ansicht etwa H-förmig ausgebildet ist.

9. Set (1) nach einem der Ansprüche 1 bis 8, **dadurch gekennzeichnet, dass** das Werkzeug (6) in stirnseitiger Ansicht spiegelsymmetrisch zu einer ersten Mittelachse (M1) und/oder spiegelsymmetrisch zu einer zweiten Mittelachse (M2) ausgebildet ist.

10. Set (1) nach einem der Ansprüche 1 bis 9, **dadurch gekennzeichnet, dass** das Berührungsvolumen durch zumindest eine, bevorzugt mehrere, Nuten in der Knochenschraube (3) gebildet ist.

11. Set (1) nach einem der Ansprüche 1 bis 10, **dadurch gekennzeichnet, dass** an einer Hülse, insbesondere einer Führungshülse, eine erste Markierung angebracht ist und hierzu eine weitere Markierung korrespondiert, welche an einem Einschraubmittel für die Knochenschraube (3) und/oder der Knochenschraube (3) angebracht ist, wobei bei fluchtender Ausrichtung der Markierungen das Berührungsvolumen für die Stellschraube (4) und die Knochenschraube (3) ausgerichtet ist.

12. Set (1) nach einem der Ansprüche 1 bis 11, **dadurch gekennzeichnet, dass** die Stellschraube (4) schräg zur Knochenschraube (3) durch die Öffnung (5) geführt ist, um im Berührungsvolumen einzugreifen, wobei ein Winkel zwischen der Stellschraube (4) und dem Knochennagel (2) 100° bis 140°, vorzugsweise 105° bis 135°, insbesondere 110° bis 130°, beträgt.

13. Set (1) nach einem der Ansprüche 1 bis 12, **dadurch gekennzeichnet, dass** die Stellschraube (4) ein Außengewinde aufweist und in der Öffnung (5) ein Innengewinde zur Aufnahme des Außengewindes der Stellschraube (4) vorgesehen ist.

14. Werkzeug (6) für ein Set (1) zur Versorgung von Knochenbrüchen, insbesondere ein Set (1) nach einem der Ansprüche 1 bis 13, insbesondere proximalen Femurfrakturen, wobei das Set (1) einen Knochennagel (2), eine Knochenschraube (3) und eine Stellschraube (4) umfasst, wobei die Knochenschraube (3) und die Stellschraube (4) durch eine Öffnung (5) des Knochennagels (2) führbar sind, um in einem Berührungsvolumen miteinander zu interagieren, **dadurch gekennzeichnet, dass** das Werkzeug (6) in einer stirnseitigen Ansicht eine Führungsöffnung (13) aufweist, sodass das Werkzeug (6) auf ein Befestigungsinstrument für die Knochenschraube (3) wie einen Dreher (9) aufbringbar ist.

15. Werkzeug (6) nach Anspruch 14, **dadurch gekennzeichnet, dass** in der Führungsöffnung (13) innenseitig zumindest eine Nase (10), vorzugsweise mehrere Nasen (10) angeordnet sind.

16. Werkzeug (6) nach Anspruch 14 oder 15, **dadurch gekennzeichnet, dass** das Werkzeug (6) in einer stirnseitigen Ansicht U-förmig oder vorzugsweise H-förmig ausgebildet ist.

17. Kombination aus einem Werkzeug (6) nach einem der Ansprüche 14 bis 16 und einem Befestigungsinstrument für eine Knochenschraube (3), wobei das Befestigungsinstrument eingerichtet ist, das Werkzeug (6) im Bereich der Führungsöffnung (13) formschlüssig aufzunehmen.

18. Kombination nach Anspruch 17, **dadurch gekennzeichnet, dass** das Werkzeug (6) am Befestigungsinstrument linear verschiebbar lagerbar oder gelagert ist.
